# EUROPEAN PATENT APPLICATION

(11) **EP 4 563 079 A1**
(43) Date of publication of application: **04.06.2025**
(21) Application number: 24211175.5
(22) Date of filing: 06.11.2024
(51) Int. Cl.: A61B 5/00, A61B 5/024, A61B 5/021, A61B 5/0205

(54) **SMART MEDICAL BRACELET WITH TRIAGE FUNCTION AND APPLICATION METHOD THEREOF**

(30) Priority: 30.11.2023 TW 112146641
(71) Applicant: iRobot Medicine Technology CO., LTD., Taipei City 114 (TW)
(72) Inventor: MI, Hsin-Wu, 114 Taipei City (TW); LIN, Tsung-Han, 114 Taipei City (TW); TU, Chang-Hung, 114 Taipei City (TW); LIAO, En-Chih, 114 Taipei City (TW)
(74) Representative: Casalonga

(57) **Abstract**

A smart medical bracelet with triage function (1) comprise: a main body (2), comprising a housing (29) with an internal accommodation space; a touch display panel (23) positioned at a top opening of the housing (29); an optical transceiver (36) situated at a first bottom opening of the housing (29); a body temperature detector (37) located at a second bottom opening of the housing (29); a pair of local charging terminals placed on one side of the housing (29); a full-color light-emitting diode (22); and a strap (4) connected to the main body (2). The strap (4) includes a flexible light-guide (41) positioned on the outer surface of the strap (4) and optically coupled to the full-color light-emitting diode (22) to guide its emitted light. The smart medical bracelet with triage function (1) is designed to detect a user's instant vital signs and aid in medical procedures.

## Description

The present invention relates to a medical bracelet, in particular to a smart medical bracelet with triage function and application method thereof, which is configured to automatically detect a user's vital signs and has a strap emitting colorful lights so as to display the user's triage status. Accordingly, the smart medical bracelet with triage function is endowed with a functionality that the user's triage status can be seen directly. A location of the smart medical bracelet with triage function can also be located in real time by a wireless positioning system. The smart medical bracelet with triage function can be applied in a hospital's various medical environments and assist in automating relevant processes.

There are currently a number of smart wearable devices on the market, but they still need to be improved to realize their applications in hospital medical environments.

In modern hospitals with emergency units, emergency medical staff face a variety of emergency patients every day; as a result, they have difficulties to immediately track down changes of patients' conditions in real time, and thus they work under enormous pressures. Since the conditions of emergency patients are diverse and changing, degrees of urgencies are diverse and may be changed at any time; the emergency medical staff must triage the patients in minimum possible time and provide correct treatments according to the urgencies of the conditions of the emergency patients; all of the above actions and processes are competing against time while life-saving rescues are being carried out in such ever-changing situations.

Therefore, how to improve the current smart wearable devices so that it can help to improve the triage procedure of the emergency unit and intelligent automation of real-time reporting of the patient's conditions and thereby these urgent problems in the emergency medical system need to be addressed: integrating emergency patient flow and triage information more effectively, assisting multi-party information integration and transmission among hospital units, medical staff, and patients, and facilitating procedures such as admission, diagnosis, treatment, and discharge. Improvements of the abovementioned issues will allow medical staff to effectively control processes, provide optimal immediate treatments for many patients, and swiftly and accurately complete various hospital tasks, even with limited medical manpower. Additionally, such improvements will help reduce workloads for relevant units and medical staff, and alleviates emergency patients' anxieties caused by uncertainties in hospital processes.

In view of the foregoing drawbacks and deficiencies of the current technology, the present invention provides a smart medical bracelet with triage function, comprising:
a main body comprising:
a housing with an internal accommodation space, and a surface featuring a top opening, a first bottom opening, a second bottom opening, a first side opening, and a second side opening;
a touch display panel positioned at the top opening of the housing, with its display surface exposed on the surface of the housing;
an optical transceiver, positioned at the first bottom opening of the housing, with its optical transceiving surface exposed on the surface of the housing;
a body temperature detector, located at the second bottom opening of the housing, with its body temperature detecting surface exposed on the surface of the housing;
a pair of local charging terminals, situated at the second side opening of the housing and exposed on the surface of the housing;
a full-color light emitting diode;
a strap connected to the main body, and the strap comprising:
   a flexible light-guide, positioned on an outer surface of the strap, optically coupled with the full- color light emitting diode to guide the emitted light.

In one embodiment, the main body additionally comprises:
a circuit board situated at the bottom of the internal accommodation space of the housing;
an upper surface of the circuit board being equipped with a battery connector and a circuit board connector;
a lower surface of the circuit board being equipped with the optical transceiver and the body temperature detector.

In another embodiment, the circuit board is also equipped with a system chip, which is electrically connected to the touch display panel, the body temperature detector, and the full-color light-emitting diode.

The present invention further provides a method of application for a smart medical bracelet with triage function, which is utilized with the smart medical bracelet with triage function, in this method
when the smart medical bracelet with triage function is attached to a user's wrist, the smart medical bracelet with triage function detects user's instant vital sign including body temperature through a body temperature detector or an optical transceiver, and transmits user's instant vital sign to an external system via a wireless communication interface; and
the smart medical bracelet with triage function communicates with the external system through another wireless communication interface to pair with a health-insurance card, and when the smart medical bracelet with triage function is paired with the health-insurance card, it is configured to authorize access to the user's medical records corresponding to the health insurance card; and
the smart medical bracelet with triage function is configured to emit lights of various colors corresponding to respective medical assessment results.

Since the smart medical bracelet with triage function has authority to access the medical records in the medical system and provide a user's instant vital signs, thereby it can assist in expediting a doctor's assessment of a patient, and the smart medical bracelet with triage function can automatically adjust the displayed light color of the multiple full-color LEDs and the flexible light-guide, based on the results of the assessment; as a result, medical staff can recognize the patient's condition by intuitively inspecting the light color, saving time and efforts and preventing misjudgments; the smart medical bracelet with triage function can inform a patient about follow-up treatment and estimated waiting times; the smart medical bracelet with triage function can also assist the patient with payment procedures, medication retrieval, and hospital discharge., and thereby it fulfills the objectives of the present invention by enhancing the automation of the emergency unit, and facilitating effective information communications among the hospital, the medical staff, administrative staff and emergency patients. This ensures seamless linkage and execution of admission, diagnosis, medical and discharge processes. Moreover, it enables hospitals, physicians, and emergency patients to swiftly and accurately complete various hospital procedures, thereby reducing the burden on administrative and medical staff and alleviating anxieties among emergency patients caused by uncertainties in hospital processes.

In order to clarify the objectives, features, and advantages of the present invention and to enhance understanding, the following embodiments are described in detail, accompanied by the corresponding drawings.

### IN THE DRAWINGS:

Figure 1 illustrates a schematic diagram depicting the appearance and structure of a smart medical bracelet with triage function of the present invention;
Figure 2 presents a front structural diagram of the main body of the smart medical bracelet with triage function of the present invention;
Figure 3 depicts a side cross-sectional structural diagram of the main body of the smart medical bracelet with triage function of the present invention;
Figure 4 illustrates a functional block diagram of the smart medical bracelet with triage function of the present invention; and
Figure 5 shows a schematic diagram of charging the smart medical bracelet with triage function of the present invention.

The technical contents, features and effects of the present invention will be clearly elucidated in the following detailed description of the preferred embodiment, accompanied by reference to the drawings. Additionally, the directional terms mentioned in the following embodiments, such as: up, down, left, right, front, back, bottom, top, etc. are only relative directions based on the drawings and do not denote absolute directional positions; therefore, the directional terms are used solely for illustrating their relative positional relationships and do not impose limitations on the present invention.

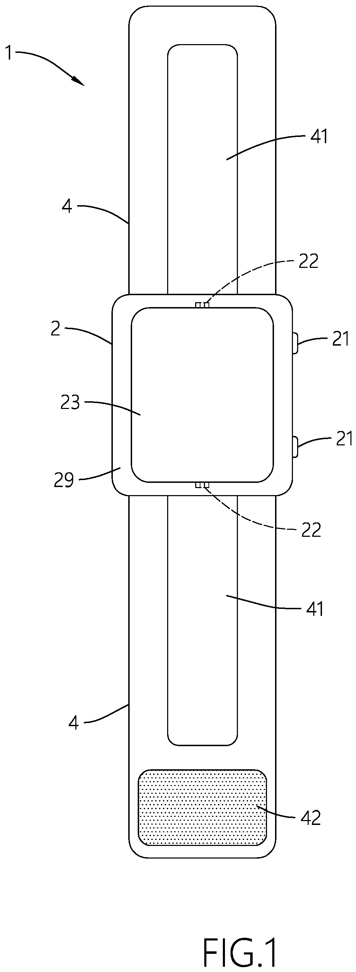

Please refer to Figure 1. Figure 1 illustrates the structural appearance of the smart medical bracelet with triage function 1 of the present invention. It comprises a main body 2, which consists of a housing 29 containing an internal accommodation space. The surface of the main body 2 features a top opening, a first bottom opening, a second bottom opening, a first side opening, and a second side opening. Additionally, the main body 2 includes a touch display panel 23 positioned on the top opening of the housing 29, as well as multiple full-color light-emitting diodes (hereinafter referred to as full-color LEDs) 22 and several buttons 21 for operating the smart medical bracelet with triage function 1. Furthermore, the smart medical bracelet with triage function 1 incorporates a strap 4 connected to the main body 2. The outer surface of the strap 4 is equipped with a hook-and-loop fastener 42 and a flexible light-guide 41, which is in contact with and optically coupled to the plurality of full-color LEDs 22. The hook-and-loop fastener 42 secures the strap 4 to the user's wrist, while the flexible light-guide 41 expands and extends the emitted light from the plurality of full-color LEDs 22 to the outer surface of the strap 4. This allows the light from the plurality of full-color LEDs 22 to be vividly and softly displayed, enabling nearby individuals to easily discern the user's vital signs status from the smart medical bracelet with triage function 1 through the light colors displayed by the flexible light-guide 41. The touch display panel 23 may consist of a thin-film transistor (TFT) liquid crystal touch display panel, a quantum dot (QD) touch display panel, or an active organic light-emitting diode (AMOLED) touch display panel, capable of information display, data input, or option selection. The flexible light-guide 41 can be crafted from soft, translucent silicone or transparent rubber capable of guiding light.

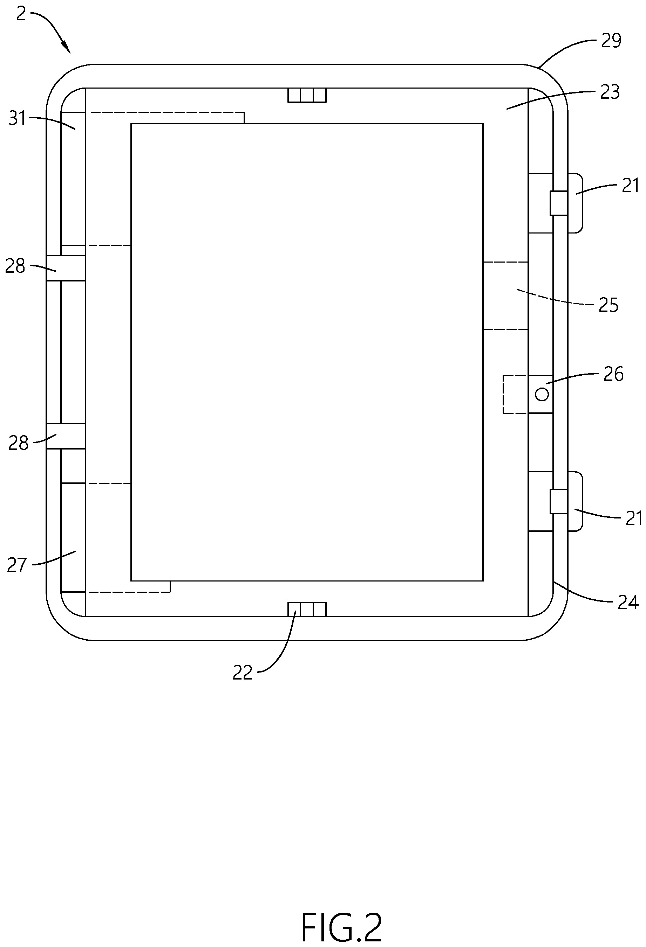

Referring to FIG. 2, it illustrates the front structure of the main body 2 of the smart medical bracelet with triage function 1 of the present invention. The plural full-color LEDs 22 are in contact with and optically coupled to the flexible light-guide 41 to guide the emitted light into it. The plural buttons 21 are positioned at the first side opening of the housing 29, while a local charging terminal pair 28 is situated at the second side opening of the housing 29.

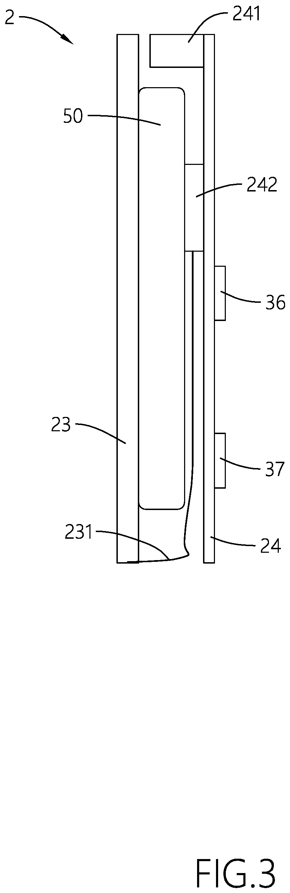

Referring to FIG. 3, it presents a side section structure of the main body 2 of the smart medical bracelet with triage function 1 of the present invention. Although the housing 29 is not depicted, a circuit board (hereinafter referred to as PCBA) 24 is placed on the bottom inner surface of the housing 29. The upper surface of the circuit board 24 hosts a battery connector 241 and a circuit board connector 242. On the lower surface of the circuit board 24, an optical transceiver 36 and a body temperature detector 37 are situated. The optical transceiver 36 is positioned at the first bottom opening of the housing 29, with its optical transceiving surface exposed on the surface of the housing 29. Similarly, the body temperature detector 37 is located at the second bottom opening of the housing 29, with its body temperature detecting surface exposed on the surface of the housing 29. The circuit board 24 may be either a flexible circuit board or an ordinary circuit board, and the body temperature detector 37 may be an infrared body temperature detector or other types of body temperature detectors.

There is an accommodation space between the circuit board 24 and the touch display panel 23 to house a battery 50. A panel flat flexible cable 231 connects the touch display panel 23 to a circuit board connector 242, facilitating power provision, data display, and control signal transmission. Furthermore, the circuit board 24 receives touch signals from the touch display panel 23.

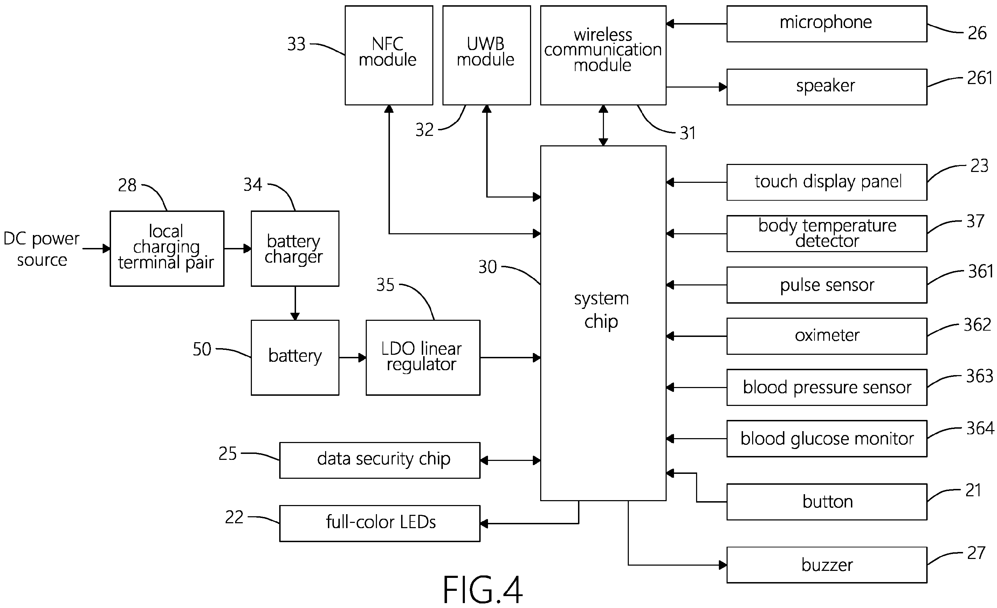

Referring to FIG. 4, it illustrates a functional block diagram of the smart medical bracelet with triage function of the present invention. A system chip 30 is positioned on the circuit board 24 and is electrically connected to various functional components for controlling and transmitting data between them. These functional components include the plural buttons 21, plural full-color LEDs 22, touch display panel 23, wireless communication module 31, ultra-wideband (UWB) module 32, near field communication (NFC) module 33, data security chip 25, buzzer 27, pulse sensor 361, oximeter 362, blood pressure sensor 363, blood glucose monitor 364, and body temperature detector 37. The touch display panel 23 is connected to the circuit board 242 and the circuit board 24 via the panel flat flexible cable 231, and then linked to the system chip 30, which may be a microcontroller (MCU). In one embodiment, the wireless communication module 31 may comprise a Wi-Fi module or a Bluetooth module, or both.

The system chip 30 also interfaces with the optical transceiver 36, controlling its operation. The optical transceiver 36, which comprises a light-emitting diode and a light sensor (not depicted), emits light configured to irradiate the skin at the user's wrist. The light sensor detects changes in light intensity as the light penetrates the skin and is reflected back. The pulse sensor 361, oximeter 362, blood pressure sensor 363, and blood glucose monitor 364 utilize the changes in light intensity sensed by the optical transceiver 36 to deduce vital signs such as heartbeat, blood oxygen, blood pressure, and blood glucose levels. In various embodiments, each of these sensors may be implemented as hardware circuits or software programs executed by the system chip 30.

The body temperature detector 37 is designed to gauge the temperature of the user's wrist skin to determine their body temperature. This detector can be an infrared body temperature detector or other types of body temperature sensors.

The main body 2 of the smart medical bracelet with triage function 1 of the present invention is designed to be externally connected to a DC power source via the local charging terminal pair 28. These terminals are linked to a battery charger 34 located within the internal accommodation space of the housing 29. The battery charger 34 is then connected to the battery connector 241, which in turn is connected to the battery 50 for charging. Similarly, the battery 50 is linked to a low dropout linear regulator (LDO) 35 through the battery connector 241. This regulator supplies power to the circuit board 24, supporting all circuit units in the main body 2, including the system chip 30, touch display panel 23, full-color light-emitting diodes 22, and other circuit components.

The buzzer 27 is programmed to audibly alert the user of the smart medical bracelet with triage function 1. It emits sound to remind the user of new messages received on the touch display panel 23 or when the user needs to respond to a message.

The wireless communication module 31 is designed for data transmission, including sending the user's vital signs-such as heartbeat, blood oxygen, blood pressure, blood glucose, and body temperature-measured by the pulse sensor 361, oximeter 362, blood pressure sensor 363, blood glucose monitor 364, and body temperature detector 37, back to an external system for real-time monitoring. Additionally, it can receive messages from external sources for the user's reference. Data output from the wireless communication module 31 is encrypted and data received by it is decrypted through the data security chip 25. Furthermore, the wireless communication module 31 connects to a microphone 26 and a speaker 261, enabling voice communications between it and matched external wireless communication modules. In one embodiment, the ultra-wideband (UWB) module 32 is used to locate the smart medical bracelet with triage function 1. For instance, with six base stations covering an area of 1800 square meters, the positioning accuracy can reach 10-30 cm. In another embodiment, the near field communication (NFC) module 33 enables noncontact proximity identification, typically within a range of 2-5 centimeters.

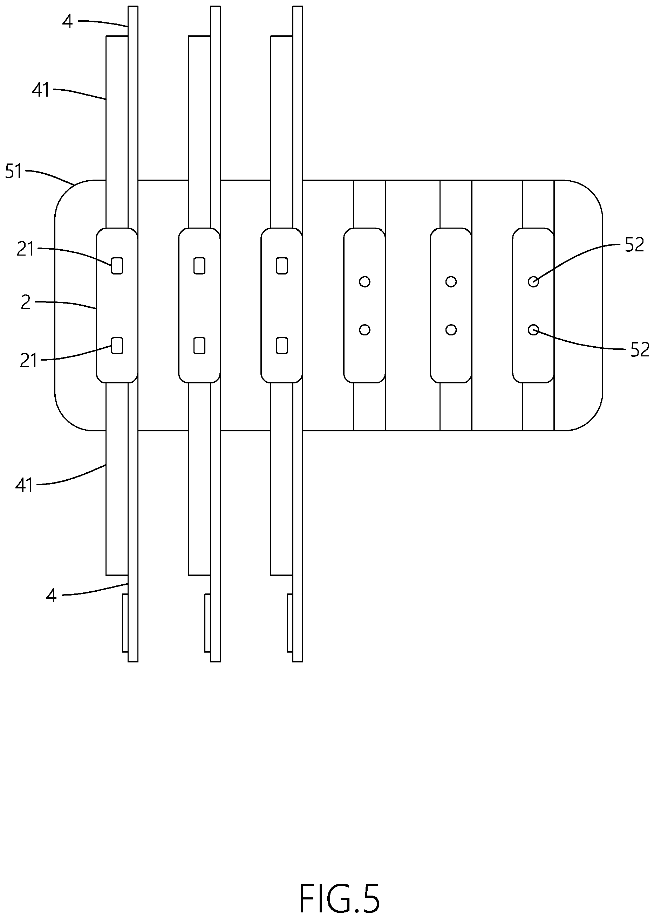

Referring to FIG. 5, it illustrates a side charging configuration of the smart medical bracelet with triage function of the present invention. As the local charging terminal pair 28 is positioned along the side direction of the housing 29, multiple smart medical bracelets with triage function 1 can only be placed along the side direction on charging terminal pairs 52 of a charging base 51 for charging. This arrangement minimizes the gap between two adjacent smart medical bracelets with triage function 1 during charging. Consequently, charging multiple smart medical bracelets with triage function of the present invention along the side direction, as depicted in FIG. 5, reduces the size of the charging base 51, saving space and costs.

In one embodiment, when the smart medical bracelet with triage function 1 of the present invention is utilized in an emergency room environment, the user, in this case, is a patient. Upon the patient's arrival in an ambulance, medical personnel can affix the smart medical bracelet with triage function 1 to the patient's wrist. At this juncture, the smart medical bracelet with triage function 1 is designed to detect and transmit the patient's immediate vital signs-such as heartbeat, blood oxygen, blood pressure, blood sugar, and body temperature-to a system on the ambulance, which displays and records these vital signs. Following the patient's arrival at the hospital emergency room, the smart medical bracelet with triage function 1 establishes communication with an emergency room system via a near field communication module (NFC). During this interaction, the emergency room system pairs the patient's health-insurance card with the smart medical bracelet with triage function 1, using the identification code of the bracelet and the health-insurance card number.

Once the patient's health-insurance card and smart medical bracelet with triage function 1 are paired, the bracelet authorizes a doctor to access the patient's medical records directly from a medical information system (HIS), without requiring the health-insurance card. Simultaneously, the smart medical bracelet with triage function 1 provides the patient's immediate vital signs data to the doctor. Consequently, the bracelet assists the physician in accessing both the patient's medical records and instant vital signs data, facilitating a rapid assessment of the patient's immediate triage status. This assessment serves as the basis for subsequent medical procedures and priorities.

Subsequently, based on the patient's immediate triage status, the emergency room system automatically adjusts the colors of the light emitted by the plurality of full-color LEDs 22 and the flexible light-guide 41 of the smart medical bracelet with triage function 1. For instance, red light signifies the most critical condition, followed by orange, blue, and green lights, with green indicating the mildest condition. The intuitive visual inspection of the light color emitted by the smart medical bracelet with triage function 1 allows medical staff to easily discern the patient's triage status, thereby saving significant labor and time in determining the patient's triage status during subsequent medical procedures. Moreover, it prevents medical staff from missing critical opportunities to treat a patient due to a lack of immediate awareness of changes in the patient's vital signs.

In one embodiment, the smart medical bracelet with triage function 1 is equipped with an ultra-wideband (UWB) module 32, enabling the hospital to pinpoint the patient's current location using a positioning system comprising multiple UWB base stations. For example, this positioning system calculates the smart medical bracelet with triage function 1's current location by measuring the signal propagation delay times of the same UWB signal received from the bracelet at each UWB base station. This capability prevents situations where medical staff are unable to locate the patient.

Additionally, in one embodiment, the time and location of a doctor's diagnosis, treatment, or instrumental examination are not only displayed on the touch display panel 23 of the smart medical bracelet with triage function 1 but also relayed to the patient through buzzing from the buzzer 27 or a voice message from the wireless communication module 31 of the bracelet. When the patient needs assistance or wishes to make a request, they can notify medical staff through the wireless communication module 31 of the smart medical bracelet with triage function 1.

Moreover, the smart medical bracelet with triage function 1 is configured to facilitate various processes for the patient. It can communicate with an external payment machine via the near field communication (NFC) interface to assist in payment processes. It can also communicate with an external intelligent medicine receiving cabinet through the NFC module to help with medicine collection. Furthermore, it can communicate with an external discharge machine via the NFC module to disconnect the pairing between the patient's health-insurance card and the smart medical bracelet with triage function 1, aiding in the hospital discharge process.

While the present invention has been described above in a preferred embodiment, it is not intended to limit the invention. Those skilled in the art may make changes and modifications without departing from the spirit and scope of the invention. Therefore, the scope of protection of the invention shall be determined by the appended patent claims.

## Claims

1. A smart medical bracelet with triage function, **characterized by** comprising:
a main body (2), comprising:
a housing (29) with an internal accommodation space, and a surface of the housing (29) comprising a top opening, a first bottom opening, a second bottom opening, a first side opening, and a second side opening;
a touch display panel (23) positioned at the top opening of the housing (29), with a display surface of the touch display panel (23) exposed on the housing's (29) surface;
an optical transceiver (36) located at the first bottom opening of the housing (29), with an optical transceiving surface of the optical transceiver (36) exposed on the housing's (29) surface;
a body temperature detector (37) situated at the second bottom opening of the housing (29), with a body temperature detecting surface of the body temperature detector (37) exposed on the housing's (29) surface;
a local charging terminal pair (28) placed at the second side opening of the housing (29) and exposed on the housing's (29) surface;
a full-color light emitting diode (22);
a strap (4) connected to the main body (2), including:
a flexible light-guide (41) on an outer surface of the strap (4), optically coupled with the full-color light emitting diode (22) to guide emitted light.

2. The smart medical bracelet with triage function as claimed in claim 1, wherein
the main body (2) additionally comprises:
a circuit board (24) positioned at a bottom of the internal accommodation space of the housing (29);
an upper surface of the circuit board (24) comprising a battery connector (241) and a circuit board connector (242);
a lower surface of the circuit board (24) hosting the optical transceiver (36) and the body temperature detector (37).

3. The smart medical bracelet with triage function as claimed in claim 2, wherein
the circuit board (24) also includes a system chip (30), electrically connected to the touch display panel (23), the body temperature detector (37), and the full-color light emitting diode (22).

4. The smart medical bracelet with triage function as claimed in claim 3, wherein
the circuit board (24) also integrates a wireless communication module (31), an ultra-wideband (UWB) module (32), or a near field communication (NFC) module (33), with the system chip (30) electrically connected to these modules.

5. The smart medical bracelet with triage function as claimed in claim 3, wherein
the optical transceiver (36) comprises a light-emitting diode and a light sensor, where light from the light-emitting diode irradiates a user's wrist skin, and the sensor detects changes in light intensity penetrating and reflecting back from the skin;
the circuit board (24) also hosts at least one vital sign detector, electrically connected to the system chip (30) and the optical transceiver (36), which calculates vital sign values based on the detected light intensity changes.

6. The smart medical bracelet with triage function as claimed in claim 3, wherein
the optical transceiver (36) includes a light-emitting diode and a light sensor, where light from the light-emitting diode irradiates a user's wrist skin, and the sensor detects changes in light intensity penetrating and reflecting back from the skin;
the system chip (30), electrically connected to the optical transceiver (36), executes at least one vital sign detection program, calculating vital sign values based on the detected light intensity changes.

7. The smart medical bracelet with triage function as claimed in claim 3, wherein the circuit board (24) also incorporates a buzzer (27) and a button (21), both electrically connected to the system chip (30), with the button (21) positioned at the first side opening of the housing (29) and exposed on the housing's (29) surface.

8. The smart medical bracelet with triage function as claimed in claim 5, wherein the vital sign detector comprises a pulse sensor (361), an oximeter (362), a blood pressure sensor (363), or a blood glucose monitor (364).

9. The smart medical bracelet with triage function as claimed in claim 6, wherein the vital sign detector comprises a pulse sensor (361), an oximeter (362), a blood pressure sensor (363), or a blood glucose monitor (364).

10. An application method of a smart medical bracelet with triage function, which is applied to the smart medical bracelet with triage function (1) as claimed in claim 1, wherein when attached to a user's wrist, the smart medical bracelet with triage function (1) detects the user's instant vital signs including body temperature through a body temperature detector (37) or an optical transceiver (36), and transmits detected data to an external system via a wireless communication interface.

11. The application method of a smart medical bracelet with triage function as claimed in claim 10, wherein the smart medical bracelet with triage function (1) communicates with the external system through another wireless communication interface to pair with a health-insurance card, and upon pairing, authorizes retrieval of the user's medical records corresponding to the health-insurance card.

12. An application method of a smart medical bracelet with triage function, which is applied to the smart medical bracelet with triage function (1) as claimed in claim 1, wherein the smart medical bracelet with triage function (1) pairs with a health-insurance card via a wireless communication interface, and upon pairing, emits lights of various colors corresponding to prospective medical assessment results.

13. The smart medical bracelet with triage function as claimed in claim 4, wherein a positioning system comprising a plurality of ultra-wideband (UWB) base stations calculates a current position of the smart medical bracelet with triage function (1) by detecting signal propagation delay times from each of the UWB base stations receiving a same UWB signal from the bracelet (1).

14. The smart medical bracelet with triage function as claimed in claim 1, wherein the bracelet (1) receives a message through a wireless communication interface and displays the message on a display panel of the bracelet (1).

15. The smart medical bracelet with triage function as claimed in claim 1, wherein the bracelet (1) communicates with an external payment machine through a wireless communication interface to make payments.

16. The smart medical bracelet with triage function as claimed in claim 1, wherein the bracelet (1) communicates with an external smart medicine receiving cabinet through a wireless communication interface to receive medicine.

17. The smart medical bracelet with triage function as claimed in claim 1, wherein upon pairing with a health-insurance card, the bracelet (1) communicates with an external returning machine through a wireless communication interface to disconnect the pairing between the card and the bracelet (1).
